Europäisches Patentamt

European Patent Office

Office européen des brevets

(11) Veröffentlichungsnummer: **0 011 745**
**B1**

(12) **EUROPÄISCHE PATENTSCHRIFT**

(45) Veröffentlichungstag der Patentschrift:
**30.12.81**

(51) Int. Cl.³: **A 61 K 9/08, A 61 K 37/22**

(21) Anmeldenummer: **79104378.9**

(22) Anmeldetag: **08.11.79**

(54) **Kolloide wässrige Zusammensetzung für pharmazeutische Zwecke, und deren Herstellung.**

(30) Priorität: **21.11.78 CH 11921/78**

(43) Veröffentlichungstag der Anmeldung:
**11.06.80 Patentblatt 80/12**

(45) Bekanntmachung des Hinweises auf die Patenterteilung:
**30.12.81 Patentblatt 81/52**

(84) Benannte Vertragsstaaten:
**AT BE CH DE FR GB IT LU NL SE**

(56) Entgegenhaltungen:
**FR-A-2 102 537**
**FR-A-2 270 887**
**FR-A-2 367 769**
**LU-A-28 584**
**LU-A-29 060**
**CHEMICAL ABSTRACTS, Band 53, Nr.7,**
**10.4.1959, Spalte 6321b.**
**Columbus, Ohio, USA,**
**O. A. ROHOLT et al.: «L- -(Dicaproyl)**

(73) Patentinhaber: **F. HOFFMANN-LA ROCHE & CO.**
**Aktiengesellschaft, CH-4002 Basel (CH)**

(72) Erfinder: **Schmidt, Dieter, Dr., Redingstrasse 20,**
**CH-4052 Basel (CH)**

(74) Vertreter: **Lederer, Franz, Dr. et al, Patentanwälte Dr.**
**Franz Lederer Relner F. Meyer Lucile-Grahn-Strasse 22,**
**D-8000 München 80 (DE)**

(56) Entgegenhaltungen:
**lecithin, a water-soluble substrate**
**for lecithinase A and D»**
**CHEMICAL ABSTRACTS, Band 56, Nr. 2,**
**22.1.1962, Spalte 1761h.**
**Columbus, Ohio, USA,**
**O. A. ROHOLT et al.: «The use of dihexanoyllecithin and**
**other lecithins as substrates for phospholipase A. With**
**addendum on aspects of micelle properties of dihex-**
**anoyllecithin»**

ACTORUM AG.

## Kolloide wässrige Zusammensetzung für pharmazeutische Zwecke, und deren Herstellung

Die vorliegende Erfindung betrifft kolloide wässrige Zusammensetzungen, sowie Verfahren zu deren Herstellung.

Es ist bekannt, wasserunlösliche Arzneimittel mittels Lecithinen zu solubilisieren (LU-A-28 584 und FR-A 2 367 769). Bei den dafür vorgeschlagenen Lecithinen handelt es sich um solche, die langkettige Fettsäurereste enthalten. Die Stabilität und damit die Lagerfähigkeit solcher Liposomenlösungen, die infolge ihrer Teilchengrösse gewöhnlich trüb oder opaleszierend sind, ist jedoch beschränkt, die die Partikel mit der Zeit zu grösseren Gebilden aggregieren. Die Aktualität dieses Missstandes wird z.B. durch die BE-A 869 551 dokumentiert, in der vorgeschlagen wurde, die Liposomenlösungen zu entwässern, um die Stabilitätsprobleme zu lösen.

In der DOS 2 730 570 sind Injektionslösungen auf wässriger Basis, welche Micellbildner, lipoide Substanzen, sowie in Wasser schwer lösliche oder unlösliche Pharmaka enthalten, beschrieben. Die als Micellbildner angegebenen Gallensäure-Derivate können jedoch eine choleretische Wirkung zeigen, welche bei gewissen Anwendungsformen unerwünscht in Erscheinung treten könnte.

Kurzkettige Lecithine d.h., solche mit niederen Acylresten, wie Dihexanoyllecithin lösen sich in Wasser zu klaren Micellen auf, und sie sind auch in der Lage, einen gewissen, jedoch in vielen Fällen zu geringen Anteil eines in Wasser wenig löslichen oder unlöslichen pharmazeutischen Wirkstoffes zu solubilisieren. Ausserdem können reine Dihexanoyllecithin-Micellen noch hämolytische Eigenschaften aufweisen.

Die Aufgabe der Erfindung bestand darin, Vehikel zur Solubilisierung von in Wasser schwer löslichen oder unlöslichen pharmazeutischen Wirkstoffen, sowie daraus herstellbare kolloide wässrige, insbesondere parenteral oder lokal verabreichbare, Zusammensetzungen zu schaffen, welche die oben genannten Nachteile nicht aufweisen.

Obschon aus der Literatur [Proc. Natl. Acad. Sci. USA 73, 3862 (1976)] hervorgeht, dass verschiedene langkettige Lecithine nur dann gut untereinander mischbar sind, wenn sie sich in der C-Atomzahl möglichst wenig unterscheiden, wurde versucht, Mischmicellen aus dem oben genannten kurzkettigen Lecithin, d.h. Dihexanoyllecithin, und einem langkettigen Lecithin, wie z.B. natürlichem Eilecithin oder Soyalecithin, herzustellen. Dabei wurde nun überraschenderweise gefunden, dass man dadurch stabile Mischmicellen erhält, welche nicht nur nicht hämolytisch wirken sondern auch in der Lage sind, beträchtlich grössere, d.h. brauchbare, Mengen eines in Wasser schwer löslichen oder unlöslichen Wirkstoffes zu solubilisieren.

Demzufolge betrifft die vorliegende Erfindung eine kolloide wässrige Zusammensetzung, welche Dihexanoyllecithin, sowie ein Phosphatid und gegebenenfalls einen oder mehrere in Wasser schwer lösliche oder unlösliche pharmazeutische Wirkstoffe sowie gegebenenfalls in Wasser lösliche pharmazeutische Wirkstoffe und/oder pharmazeutische Hilfsstoffe enthält, wobei das Molverhältnis von Phosphatid zu Dihexanoyllecithin im Bereich von 1:3 bis 1:1 liegt.

Beispiele von Phosphatiden sind Eilecithin, Soyalecithin, Dipalmitoyllecithin, Dimyristoyllecithin oder Phosphatidylinosit. Besonders bevorzugt sind Eilecithin und Soyalecithin.

Der Gehalt an Dihexanoyllecithin plus Phosphatid in der Zusammensetzung kann über weite Grenzen variieren und kann z.B. 50–200 mg/ml betragen.

Die erfindungsgemässe kolloide wässrige Zusammensetzung kann zusätzlich einen oder mehrere in Wasser schwer lösliche oder unlösliche pharmazeutische Wirkstoffe sowie gegebenenfalls in Wasser lösliche pharmazeutische Wirkstoffe und/oder pharmazeutische Hilfsstoffe enthalten.

Als in Wasser schwer oder unlösliche pharmazeutische Wirkstoffe kommen insbesondere Benzodiazepine und fettlösliche Vitamine in Betracht. Weiterhin kommen in Wasser schwer lösliche oder unlösliche Neuroleptika, Antidepressiva, Antiinfektiva und Steroide in Frage.

Beispiele von Benzodiazepinen sind Diazepam, Clonazepam, Flunitrazepam, Medazepam und Bromazepam. Besonders bevorzugt ist Diazepam.

Beispiele von fettlöslichen Vitaminen im Rahmen der Erfindung sind die Vitamine A, D, E und K, sowie Derivate davon. Bevorzugt sind Vitamin E-Acetat und Vitamin $K_1$.

Beispiele von in Wasser löslichen pharmazeutischen Wirkstoffen sind wasserlösliche Vitamin, wie z.B. Vitamin $B_1$, Vitamin $B_6$, Vitamin $B_{12}$ und Mischungen aus zwei oder mehr der genannten Vitamine.

Beispiele für pharmazeutische Hilfsstoffe sind Antioxydantien, wie Natriumhydrogensulfit, Natriumpyrosulfit oder Vitamin E-Acetat.

Die erfindungsgemässen kolloiden wässrigen Zusammensetzungen können zusätzlich isotonisierende Zusätze, wie z.B. Kochsalz- und Glucoselösung, Tris-Puffer, Phosphat-Puffer, Citratpuffer, Glycinpuffer, Citrat-Phosphat-Mischpuffer, usw. enthalten.

Als weitere Komponente kann auch noch ein wasserlösliches, untoxisches organisches Lösungsmittel, wie Äthanol in der Zusammensetzung enthalten sein, dessen Menge für parenterale Applikationszwecke bis etwa 6%, für lokale Applikationszwecke bis etwa 40% betragen kann.

Die in Wasser schwer löslichen oder unlöslichen pharmazeutischen Wirkstoffe in der kolloiden wässrigen Zusammensetzungen eignen sich z.B. zur parenteralen oder lokalen Verabreichung.

Der Anteil des in Wasser schwer löslichen oder unlöslichen pharmazeutischen Wirkstoffes in der kolloiden wässrigen Zusammensetzung kann über weite Grenzen variieren und kann z.B. 0,1–20 mg/ml Lösung betragen.

Der osmotische Druck von Injektions- bzw. Infusionslösungen kann ebenfalls in gewissen Grenzen variieren und entspricht vorteilhafterweise annähernd demjenigen des Blutes.

Die erfindungsgemässen kolloiden wässrigen Zusammensetzungen könne durch blosses Vermischen der einzelnen Komponenten hergestellt werden. Diese Verfahrensweise kommt insbesondere bei einem niedrigen Molverhältnis von Phosphatid zu Dihexanoyllecithin (z.B. 1:3 bis 1:2) in Betracht; die zur Erzielung des homogenen Gemisches erforderliche Zeit lässt sich durch Erwärmen verkürzen.

Eine bevorzugte Ausführungsform zur Herstellung der erfingungsgemässen Zusammensetzungen besteht darin, das Dihexanoyllecithin, das Phosphatid und gegebenenfalls einen oder mehrere in Wasser schwer lösliche oder unlösliche pharmazeutische Wirkstoffe in einem organischen Lösungsmittel zu lösen, in welchem die Komponenten hinreichend löslich sind, darauf das organische Lösungsmittel abzudampfen und anschliessend Wasser oder Pufferlsöung zuzugeben.

Bei einer weiteren bevorzugten Ausführungsform werden das Dihexanoyllecithin, bis zu etwa ein Mol-Äquivalent Phosphatid und etwa 100 bis zu etwa 200 Mol-Äquivalent Wasser und gegebenenfalls ein oder mehrere in Wasser schwer lösliche oder unlösliche pharmazeutische Wirkstoffe intensiv gerührt, bis das Gemisch homogen erscheint, worauf weiteres Wasser oder Pufferlösung zugesetzt wird, bis die gewünschte Verdünnung bzw. Konzentration erreicht ist.

Die Zeit, die benötigt wird, bis das oben erwähnte Gemisch nach dem Rühren homogen erscheint, kann in der Regel durch kurzzeitiges Erwärmen oder durch Zugabe eines organischen Lösungsmittels, wie Äthanol, verkürzt werden.

Bei etwa äquimolaren Verhältnissen Phosphatid:
Dihexanoyllecithin (z.B. zwischen 0,9:1 und 1:1) löst man zweckmässigerweise das Dihexanoyllecithin und das Phosphatid in einem geeigneten organischen Lösungsmittel, z.B. einem Alkohol, wie Äthanol, dampft das Lösungsmittel ab und gibt auf ein Mol Dihexanoyllecithin etwa 100 bis etwa 200 Mol Wasser zu. Diese Phase wird unter Erwärmen auf etwa 80 °C innert ca. 15 Minuten homogen, worauf mit Wasser oder Pufferlösung auf die gewünschte Konzentration verdünnt werden kann.

Es ist auch möglich, das Vehikel auf wässriger Basis, wie oben beschrieben, herzustellen und anschliessend den bzw. die in Wasser schwer löslichen oder unlöslichen pharmazeutischen Wirkstoffe in die kolloide wässrige Zusammensetzung einzuführen. Diese Variante kann insbesondere bei der Solubilisierung von Benzodiazepinen angewandt werden.

Die nachfolgenden Beispiele erläutern die Erfindung.

**Beispiel 1**

1,36 mMol (620 mg) Dihexanoyllecithin und 1,22 mMol (939 mg) Eilecithin [nach Singleton et al., J. Am. Oil. Chem. Soc. 42 53 (1965) isoliert] werden in ca. 30 ml Äthanol aufgelöst. Durch rasches Verdampfen am Vakuum bei etwa 30 °C erzeugt man aus dieser Lösung in einem Kolben einen Film. Dieser wird mit 1/15 M Phosphatpufferlösung zu 10 ml einer klaren wässrigen Zusammensetzung aufgelöst.

**Beispiel 2**

1 mMol (453 mg) Dihexanoyllecithin und 500 μMol (445 mg) Soyalecithin werden mit 2 ml 1/15 M Phosphatpuffer (pH 7) mittels eines Homogenisators gut durchgemischt. Nach mehrstündigem Stehenlassen bei Zimmertemperatur bildet sich daraus eine klare viskose Phase, die mit 1/15 M Phosphatpufferlösung zu 10 ml einer wässrigen Zusammensetzung aufgelöst wird.

**Beispiel 3**

Man arbeitet wie in Beispiel 2, jedoch erfolgt die Bildung der viskosen Phase durch Erwärmen auf etwa 80 °C während etwa 15 Minuten.

**Beispiel 4**

1 mMol (453 mg) Dihexanoyllecithin und 500 μMol (445 mg) Soyalecithin werden mit 10 ml 1/15 M Phosphatpufferlösung vermischt. Es wird während etwa 18 Stunden bei Raumtemperatur gerührt, worauf man eine klare wässrige Zusammensetzung erhält.

**Beispiel 5**

Man arbeitet wie im Beispiel 4, jedoch erfolgt die Solubilisierung durch Rühren bei etwa 80 °C während etwa 1 Stunde.

**Beispiel 6**

1 mMol (453 mg) Dihexanoyllecithin und 1 mMol (890 mg) Eilecithin werden in etwa 30 ml Äthanol aufgelöst. Durch rasches Verdampfen am Vakuum bei etwa 30 °C erzeugt man aus dieser Lösung in einem Kolben einen Film. Dieser wird mit etwa 3 ml 1/15 M Phosphatpufferlösung gut durchgemischt. Nach etwa einstündigem Stehenlassen bei Raumtemperatur bildet sich daraus eine klare viskose Phase, die mit weiterer Phosphatpufferlösung auf 10 ml Endvolumen verdünnt wird.

**Beispiel 7**

1,36 mMol (620 mg) Dihexanoyllecithin und 1,22 mMol (939 mg) Eilecithin werden zusammen mit 33 mg Diazepam in 30 ml Äthanol aufgelöst. Durch rasches Verdampfen am Vakuum bei etwa 30 °C erzeugt man aus dieser Lösung in einem Kolben einen Film. Dieser wird mit 1/15 M Phosphatpufferlösung zu 10 ml einer klaren Zusammensetzung aufgelöst.

**Beispiel 8**

Man arbeitet wie in Beispiel 7, jedoch erfolgt die Zugabe des Diazepams zum Vehikel am Schluss.

Beispiel 9

650 µMol (295 mg) Dihexanoyllecithin und 400 µMol (308 mg) Eilecithin werden zusammen mit 100 mg Vitamin $K_1$ in 30 ml Äthanol aufgelöst. Durch rasches Verdampfen am Vakuum bei etwa 30 °C erzeugt man aus dieser Lösung in einem Kolben einen Film. Dieser wird zunächst mit 1 ml 1/15 M Phosphatpufferlösung gemischt. Nach 2-stündigem Erwärmen auf etwa 80 °C erhält man daraus eine klare gelbe Phase, die mit weiterer Phosphatpufferlösung zu 10 ml einer klaren Zusammensetzung aufgelöst wird.

Beispiel 10

1 mMol (453 mg) Dihexanoyllecithin, 500 µMol (445 mg) Soyalecithin und 25 mg Diazepam werden mit 2 ml 1/15 M Phosphatpufferlösung (pH 7) gut durchmischt und mehrere Stunden bei Zimmertemperatur stehengelassen, bis sich eine klare viskose Phase gebildet hat. Diese wird mit weiterer Phosphatpufferlösung zu 10 ml einer klaren Zusammensetzung aufgelöst.

Beispiel 11

1 mMol (453 mg) Dihexanoyllecithin, 500 µMol (445 mg) Soyalecithin und 100 mg Vitamin $K_1$ werden mit 2 ml 1/15 M Phosphatpuffer (pH 7) gut durchmischt und dann für 2 Stunden auf etwa 80 °C erhitzt. Dabei bildet sich eine klare Phase, die etwas viskos ist. Beim Verdünnen mit weiterer Phosphatpufferlösung erhält man daraus 10 ml einer wässrigen Zusammensetzung.

Beispiel 12

Es wird wie in Beispiel 11 vorgegangen, jedoch werden anstelle des Vitamins $K_1$ 100 mg Vitamin E-Acetat eingesetzt.

Beispiel 13

5,6 mMol (2,54 g) Dihexanoyllecithin, 4,2 mMol (3,23 g) Eilecithin und 115,5 mg Diazepam werden in etwa 100 ml Äthanol aufgelöst. Durch rasches Verdampfen am Vakuum bei etwa 30 °C erzeugt man aus dieser Lösung in einem Kolben einen Film. Dieser wird mit 1/15 M Phosphatpufferlösung, welche vorher mit Stickstoff gesättigt worden war, zu 35 ml einer klaren wässrigen Zusammensetzung aufgelöst. Die wässrige Zusammensetzung wird anschliessend unter Laminar-flow-Bedingungen steril filtriert und in Stickstoff enthaltende 3 ml Ampullen abgefüllt. Diese werden zugeschmolzen. Während der ganzen Prozedur wird Luftkontakt vermieden, um eine sauerstofffreie Lösung in der Ampulle zu erhalten.

Beispiel 14

2,1 mMol (951 mg) Dihexanoyllecithin, 1,4 mMol (1078 mg) Eilecithin und 350 mg Vitamin $K_1$ werden in etwa 100 ml Äthanol aufgelöst. Durch rasches Verdampfen am Vakuum bei etwa 30 °C erzeugt man aus dieser Lösung in einem Kolben einen Film. Dieser wird mit etwa 3,5 ml 1/15 M Phosphatpufferlösung und etwa 0,7 ml Äthanol intensiv durchmischt. Nach viertägigem Stehenlassen bei Raumtemperatur bildet sich daraus eine klare, viskose Phase, die mit weiterer 1/15 M Phosphatpufferlösung, die mit Stickstoff gesättigt worden war, zu 35 ml einer klaren, gelben wässrigen Zusammensetzung verdünnt wird. Unter Laminar-Bedingungen wird die wässrige Zusammensetzung steril filtriert und in Stickstoff enthaltende 1 ml Ampullen abgefüllt. Während der ganzen Prozedur wird Luftkontakt vermieden, um eine sauerstofffreie Ampulle zu erhalten.

Beispiel 15

3,6 mMol (1430 mg) Dibutyryllecithin, 0,6 mMol (272 mg) Dihexanoyllecithin und 1,2 mMol (924 mg) Eilecithin werden zusammen mit 33 mg Diazepam in 30 ml Äthanol aufgelöst. Durch rasches Verdampfen am Vakuum bei etwa 30 °C erzeugt man aus dieser Lösung in einem Kolben einen Film. Dieser wird mit 1/15 M Phosphatpufferlösung zu 10 ml einer klaren Zusammensetzung aufgelöst.

Beispiel 16

1,0 mMol (453 mg) Dihexanoyllecithin, 1,5 mMol (1,335 g) Soyalecithin und 5 mg 9-(4-Methoxy-2,3,6-trimethylphenyl)-3,7-dimethyl-nona-2,4,6,8-tetraen-1-säure-äthylamid werden in 4 ml Äthanol aufgelöst. Unter Rühren verdünnt man dann langsam mit 1/15 M Phosphatpuffer auf 10 ml.

**Patentansprüche**

1. Kolloide wässrige Zusammensetzung, welche Dihexanoyllecithin sowie ein Phosphatid und gegebenenfalls einen oder mehrere in Wasser schwer lösliche oder unlösliche pharmazeutische Wirkstoffe, sowie gegebenenfalls einen oder mehrere in Wasser lösliche pharmazeutische Wirkstoffe und/oder pharmazeutische Hilfsstoffe enthält, wobei das Molverhältnis von Phosphatid zu Dihexanoyllecithin im Bereich von 1:3 bis 1:1 liegt.

2. Zusammensetzung nach Anspruch 1, dadurch gekennzeichnet, dass das Phosphatid Eilecithin, Soyalecithin, Dipalmitoyllecithin, Dimyristoyllecithin oder Phosphatidylinosit ist.

3. Zusammensetzung nach einem der Ansprüche 1 oder 2, dadurch gekennzeichnet, dass der Gehalt an Dihexanoyllecithin plus Phosphatid in der Zusammensetzung 50–200 mg/ml beträgt.

4. Zusammensetzung nach den Ansprüchen 1–3, dadurch gekennzeichnet, dass sie als pharmazeutischen Wirkstoff ein Benzodiazepin enthält.

5. Zusammensetzung nach Anspruch 4, dadurch gekennzeichnet, dass das Benzodiazepin Diazepam ist.

6. Zusammensetzung nach den Ansprüchen 1–3, dadurch gekennzeichnet, dass sie als pharmazeutischen Wirkstoff ein fettlösliches Vitamin enthält.

7. Zusammensetzung nach Anspruch 6, dadurch gekennzeichnet, dass sie als pharmazeutischen Wirkstoff Vitamin A, D, E oder K oder ein Derivat davon enthält.

8. Zusammensetzung nach den Ansprüchen 1–7, dadurch gekennzeichnet, dass der Gehalt des bzw. der in Wasser schwer löslichen oder unlöslichen pharmazeutischen Wirkstoffe(s) in der Zusammensetzung 0,1 bis 20 mg/ml beträgt.

9. Verfahren zur Herstellung einer Zusammensetzung nach Anspruch 1, dadurch gekennzeichnet, dass man die einzelnen Komponenten vermischt.

10. Variante des Verfahrens nach Anspruch 9, dadurch gekennzeichnet, dass man das Dihexanoyllecithin, das Phosphatid und gegebenenfalls einen oder mehrere in Wasser schwer lösliche oder unlösliche pharmazeutische Wirkstoffe und/ oder pharmazeutische Hilfsstoffe in einem geeigneten organischen Lösungsmittel löst, hierauf das organische Lösungsmittel abdampft und dann Wasser zugibt.

11. Variante des Verfahrens nach Anspruch 9, dadurch gekennzeichnet, dass man ungefähr ein Mol-Äquivalent Dihexanoyllecithin, bis zu etwa ein Mol-Äquivalent Phosphatid und etwa 100 bis 200 Mol-Äquivalent Wasser und gegebenenfalls einen oder mehrere in Wasser schwer lösliche oder unlösliche pharmazeutische Wirkstoffe und/ oder pharmazeutische Hilffstoffe intensiv rührt, bis das Gemisch homogen erscheint, worauf weiteres Wasser oder Pufferlösung zugesetzt wird, bis die gewünschte Verdünnung bzw. Konzentration erreicht wird.

12. Variante des Verfahrens nach einem der Ansprüche 10 oder 11, dadurch gekennzeichnet, dass man zu Beginn ohne den bzw. die in Wasser schwer löslichen oder unlöslichen pharmazeutischen Wirkstoffe arbeitet und den bzw. die pharmazeutischen Wirkstoffe erst am Schluss in die Zusammensetzung einführt.

**Revendications**

1. Composition aqueuse colloïdale qui contient de la dihexanoyllécithine ainsi qu'un phosphatide, éventuellement une ou plusieurs substances actives pharmaceutiques difficilement solubles ou insolubles dans l'eau, et éventuellement une ou plusieurs substances actives pharmaceutiques et/ ou additifs pharmaceutiques solubles dans l'eau, le rapport molaire du phosphatide à la dihexanoyllécithine étant situé dans un intervalle allant de 1:3 à 1:1.

2. Composition selon la revendication 1, caractérisée en ce que le phosphatide est de la lécithine d'œuf, de la lécithine de soya, de la dipalmitoyllécithine, de la dimyristoyllécithine ou du phosphatidylinositol.

3. Composition selon l'une des revendications 1 et 2, caractérisée en ce que la teneur en dihexanoyllécithine et phosphatide confondus dans la composition s'élève à 50–200 mg/ml.

4. Composition selon l'une des revendications 1 à 3, caractérisée en ce qu'elle contient comme substance active une benzodiazépine.

5. Composition selon la revendication 4, caractérisée en ce que la benzodiazépine est le diazépam.

6. Composition selon l'une des revendications 1 à 3, caractérisée en ce qu'elle contient comme substance active pharmaceutique une vitamine liposoluble.

7. Composition selon la revendication 6, caractérisée en ce qu'elle contient comme substance active pharmaceutique de la vitamin A, de la vitamine D, de la vitamine E ou de la vitamine K, ou encore un dérivé de ces vitamines.

8. Composition selon l'une des revendications 1 à 7, caractérisée en ce que le teneur en substance(s) active(s) pharmaceutique(s) difficilement soluble(s) ou insoluble(s) dans l'eau se situe dans la composition entre 0,1 et 20 mg/ml.

9. Procédé de préparation d'une composition selon la revendication 1, caractérisé en ce qu'on mélange entre eux les composants isolés.

10. Variante du procédé selon la revendication 9, caractérisée en ce qu'on dissout la dihexanoyllécithine, le phosphatide et éventuellement une ou plusieurs substances actives pharmaceutiques difficilement solubles ou insolubles dans l'eau et/ ou additifs pharmaceutiques dans un solvant organique approprié, puis on fait évaporer ledit solvant organique et on ajoute de l'eau.

11. Variante du procédé selon la revendication 9, caractérisée en ce qu'on agite de façon intensive environ une partie molaire de dihexanoyllécithine, au maximum environ une partie molaire de phosphatide et environ 100 à 200 parties molaires d'eau et éventuellement une ou plusieurs substances actives pharmaceutiques et/ou additifs pharmaceutiques difficilement solubles ou insolubles dans l'eau, jusqu'à ce que le mélange apparaisse homogène, puis on ajoute de l'eau supplémentaire ou une solution tampon, jusqu'à ce que l'on atteigne la dilution ou la concentration voulue.

12. Variante du procédé selon l'une des revendications 10 et 11, caractérisée en ce qu'on travaille au début sans la ou les substances actives pharmaceutiques difficilement solubles ou insolubles dans l'eau, et en ce qu'on introduit la ou les substances actives pharmaceutiques seulement à la fin dans la composition.

**Claims**

1. Colloidal aqueous composition, which contains dihexanoyl lecithin as well as a phosphatide and, if desired, one or more pharmaceutical active substances difficultly soluble or insoluble in water, as well as, if desired, one or more pharmaceutical active substances soluble in water and/or pharmaceutical adjuvant substances, whereby the molar ratio of phosphatide to dihexanoyl lecithin lies in the range of 1:3 to 1:1.

2. Composition according to claim 1, characterised in that the phosphatide is egg lecithin, soya lecithin, dipalmitoyl lecithin, dimyristoyl lecithin or phosphatidylinositol.

3. Composition according to one of claims 1 or 2, characterised in that the content of dihexanoyl lecithin plus phosphatide in the composition amounts to 50–200 mg/ml.

4. Composition according to claims 1–3, characterised in that it contains a benzodiazepine as the pharmaceutical active substance.

5. Composition according to claim 4, characterised in that the benzodiazepine is diazepam.

6. Composition according to claims 1–3, characterised in that it contains a fat-soluble vitamin as the pharmaceutical active substance.

7. Composition according to claim 6, characterised in that it contrains vitamin A, D, E or K or a derivative thereof as the pharmaceutical active substance.

8. Composition according to claims 1–7, characterised in that the content of pharmaceutical active substance(s) difficultly soluble or insoluble in water in the composition amounts to 0,1 to 20 mg/ml.

9. Process for the manufacture of a composition according to claim 1, characterised in that the individual components are mixed.

10. Variant of the process according to claim 9, characterised in that the dihexanoyl lecithin, the phosphatide and, if desired, one or more pharmaceutical active substances difficultly soluble or insoluble in water and/or pharmaceutical adjuvant substances are dissolved in a suitable organic solvent, thereupon the organic solvent is evaporated and then water is added.

11. Variant of the process according to claim 9, characterised in that approximately one mol equivalent of dihexanoyl lecithin, up to about one mol equivalent of phosphatide and about 100 to 200 mol equivalents of water and, if desired, one ore more pharmaceutical active substances difficultly soluble or insoluble in water and/or pharmaceutical adjuvant substances are intensively stirred until the mixture appears homogeneous, whereupon further water or buffer solution is added until the desired dilution or concentration is achieved.

12. Variant of the process according to one of claims 10 or 11, characterised in that the process is carried out at the beginning without the pharmaceutical active substance(s) difficultly soluble or insoluble in water and the pharmaceutical active substance(s) is/are introduced into the composition only at the end.